# FASCICULE DE BREVET EUROPEEN

(11) **EP 1 053 723 B1**
(45) Date de publication et mention de la délivrance du brevet: **07.07.2004**
(21) Numéro de dépôt: 00420097.8
(22) Date de dépôt: 18.05.2000
(51) Int. Cl.: A61F 2/34

(54) **Implant cotyloidien médialisé et à noyau mobile**
Versetztes Hüftgelenkpfannenimplantat mit beweglichem Einsatz
Shifted cotyloid implant with movable insert

(30) Priorité: 19.05.1999 FR 9906609
(43) Date de publication de la demande: 22.11.2000
(73) Titulaire: Himmer, Olivier, 5004 Bouge (BE); Laffay, Jean Pierre, 90340 Chevremont (FR); Bouxin, Bertrand, 62155 Merlimont (FR)
(72) Inventeur: Himmer, Olivier, 5004 Bouge (BE); Laffay, Jean Pierre, 90340 Chevremont (FR); Bouxin, Bertrand, 62155 Merlimont (FR)
(74) Mandataire: Thivillier, Patrick

(56) Documents cités:
- EP-A- 0 461 019
- EP-A- 0 640 325
- EP-A- 0 821 922
- DE-A- 3 602 081
- DE-A- 4 428 407
- FR-A- 2 387 641
- FR-A- 2 413 078
- FR-A- 2 735 355
- US-A- 3 813 699

## Description

L'invention se rattache au secteur technique des implants orthopédiques pour l'articulation de la hanche.

Plus particulièrement l'invention concerne un implant cotyloïdien comprenant, d'une manière connue, un insert ou noyau notamment en polyéthylène monté dans une cupule métallique. Cette cupule métallique, de forme générale hémisphérique, est impactée dans la cavité cotyloïdienne de l'os iliaque en y étant maintenue par différents moyens en combinaison ou non avec du ciment. L'insert en polyéthylène délimite une cavité interne hémisphérique destiné à recevoir, avec capacité d'articulation, la tête fémorale que présente généralement une tige destinée à être impactée dans le canal médullaire du fémur correspondant. De nombreuses formes de réalisation de ce type d'implant cotyloïdien ont été proposées.

Un des problèmes techniques majeurs que doivent résoudre ces différents types d'implants cotyloïdiens, est de supprimer, ou tout au moins de réduire, au maximum les risques de luxation.

Une solution avantageuse ressort de l'enseignement du brevet FR 2.735.355 qui se propose de corriger le décalage vers le bas du centre de rotation de l'élément prothétique dans le cas où les différentes faces, tant internes qu'externes, de l'insert polyéthylène et de la cupule métallique, sont concentriques. Une telle excentration est un facteur important d'instabilité.

Le but recherché dans ce brevet est donc de corriger le phénomène d'excentration résultant de la préparation de la cavité cotyloïdienne, en remédialisant le centre de rotation et en implantant une cupule recentrée.

Dans ce but, la surface extérieure de la cupule est délimitée par une enveloppe hémisphérique dont le centre est décalé vers l'extérieur par rapport au centre de sa surface interne, lui-même confondu avec le centre de courbure de la surface interne de la sphère et le centre de rotation de la tête fémorale. Il en résulte que l'épaisseur de la cupule métallique croit en direction de son bord annulaire externe. Cette médialisation résultant du décalage des deux centres de rotation dans l'axe de symétrie de l'implant, constitue donc une solution technique avantageuse au phénomène de luxation.

Toutefois, une telle solution est susceptible de générer un effet de came, notamment dans des cas de mouvements d'abduction, compte tenu du décalage des deux centres de rotation. En effet, si l'on considère la solution technique divulguée par l'enseignement du brevet français précité 2.735.355, l'insert en polyéthylène déborde du bord annulaire externe de la cupule métallique, d'une manière correspondant sensiblement au décalage des deux centres de rotation. En outre, l'insert en polyéthylène présente des agencements de fixation sous forme par exemple d'un système d'encliquetage par rapport à la cupule métallique, de sorte que ledit insert est rendu solidaire de la cupule, sans aucune possibilité de débattement angulaire.

En résumé, cette solution technique de médialisation offre l'avantage d'augmenter la stabilité, mais présente l'inconvénient de limiter la mobilité en générant un effet de came prématuré, par ailleurs facteur d'usure du polyéthylène, compte tenu du décalage des centres de rotation.

L'invention s'est fixée pour but de remédier à ces inconvénients, de manière simple, sûre, efficace et rationnelle.

Le problème que se propose de résoudre l'invention est de supprimer ou tout au moins de diminuer, d'une manière importante, tout effet de luxation, par une rétentivité de la tête fémorale dans un insert mobile à l'intérieur d'un implant cotyloïdien du type de celui défini dans le brevet FR 2.735.355. Le mouvement conjugué de la tête dans l'insert et de l'insert dans la cupule augmente sensiblement la stabilité sans générer de contrainte d'arrachement ni d'effet came prématuré.

Pour résoudre un tel problème, il a été conçu et mis au point un implant cotyloïdien comprenant, d'une manière connue, un noyau en polyéthylène monté dans une cupule métallique, et recevant une tête fémorale, ledit implant étant remarquable par la combinaison des caractéristiques suivantes :
- les centres de courbure de la surface interne et de la surface externe du noyau, correspondant au centre de rotation de la tête fémorale, sont confondus avec le centre de la surface interne de la cupule ;
- la surface externe de la cupule est délimitée par une enveloppe hémisphérique dont le centre est décalé vers l'extérieur par rapport au centre de la surface interne de ladite cupule ;
- le noyau est monté à l'intérieur de la cupule avec capacité de pivotement multidirectionnel et reçoit la tête fémorale avec capacité de rétention.

Compte tenu du problème posé, les différents centres sont disposés en alignement selon l'axe de symétrie du noyau et de la cupule.

Pour résoudre le problème posé d'éviter au noyau mobile d'échapper la cupule métallique, la face externe convexe de la cupule est sensiblement inférieure à une demi-sphère dont le bord circulaire ouvert présente des agencements de recouvrement partiel du noyau, quelle que soit sa position angulaire.

Avantageusement, ces agencements de recouvrement sont constitués par un rebord cylindrique formé dans le prolongement de la surface interne hémisphérique de la cupule et en retrait de sa surface externe.

Pour résoudre le problème posé d'assurer la fixation de la cupule dans la cavité cotyloïdienne de l'os iliaque, par un effet connu sous le nom de "PRESS-FIT", la face externe de la cupule délimite un angle d'environ 160°, tandis que la face interne du rebord cylindrique est situé dans un plan horizontal parallèle à celui de ladite face externe, à sensiblement 200°.

Le noyau mobile délimite une forme externe sphérique d'environ 225° et une cavité interne cylindro-sphérique compte tenu de l'effet de rétention recherché par rapport à la tête fémorale.

Un autre problème que se propose de résoudre l'invention est d'assurer une fixation sûre et efficace de la cupule dans la cavité cotyloïdienne, en évitant l'emploi des organes rapportés du type vis, tout en ayant pour objectif d'obtenir une parfaite stabilité. Dans ce but, la face externe de la cupule présente, à partir de son bord supérieur où est formé en retrait le rebord cylindrique, des agencements de positionnement ou d'extraction et de stabilité dans la cavité cotyloïdienne de l'os iliaque recevant ladite cupule.

Avantageusement, ces agencements sont constitués, d'une part, par une série de rainures circulaires parallèles fragmentées dont les bords externes constituent, en combinaison, une denture et, d'autre part, par des entailles aptes à recevoir des griffes d'outil impacteur ou extracteur.

Compte tenu de ces dispositions, selon une autre caractéristique, la cupule est pleine en délimitant une surface interne hémisphérique entièrement lisse et exempt de tout orifice, afin d'éviter toute usure prématurée du polyéthylène en considérant les mouvements multidirectionnels auxquels il peut être soumis.

Pour résoudre le problème posé d'améliorer l'ancrage de l'ensemble de l'implant, le rebord cylindrique présente des agencements externes aptes à permettre l'engagement de pions d'ancrage ou vis qui apparaissent à l'extérieur de la surface externe de la cupule.

L'invention est exposée ci-après plus en détail à l'aide des figures des dessins annexés dans lesquels :
- la figure 1 est une vue en perspective de la cupule métallique ;
- la figure 2 est une vue en coupe longitudinale de la cupule ;

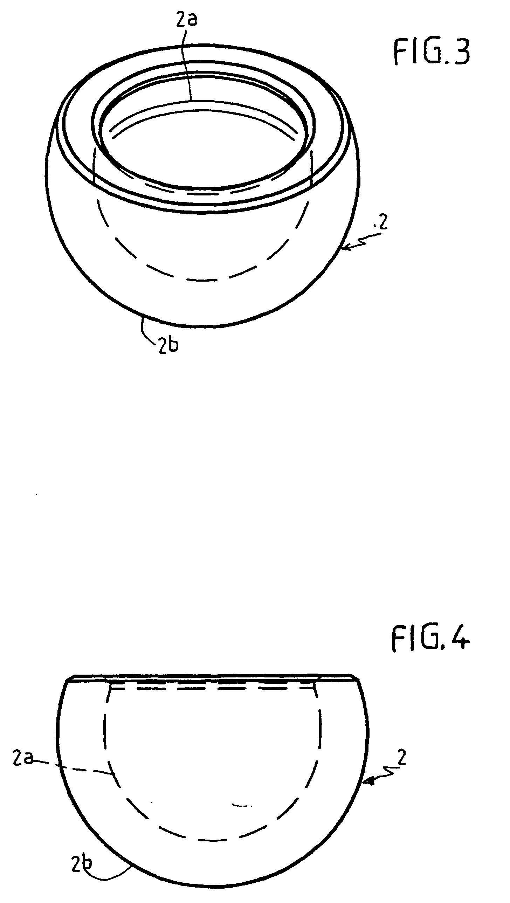

- la figure 3 est une vue en perspective du noyau ou insert en polyéthylène ;
- la figure 4 est une vue en coupe longitudinale du noyau ;

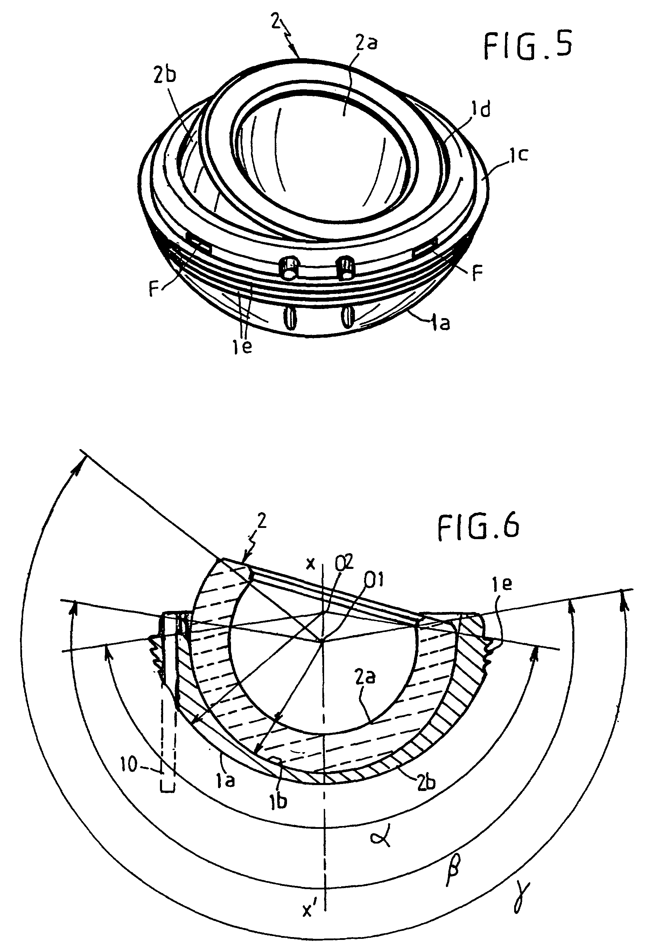

- la figure 5 est une vue en perspective de la cupule recevant le noyau ;
- la figure 6 est une vue en coupe longitudinale correspondant à la figure 5 ;
- les figures 7, 8 et 9 sont des vues de face montrant différentes orientations du noyau par rapport à la cupule ;

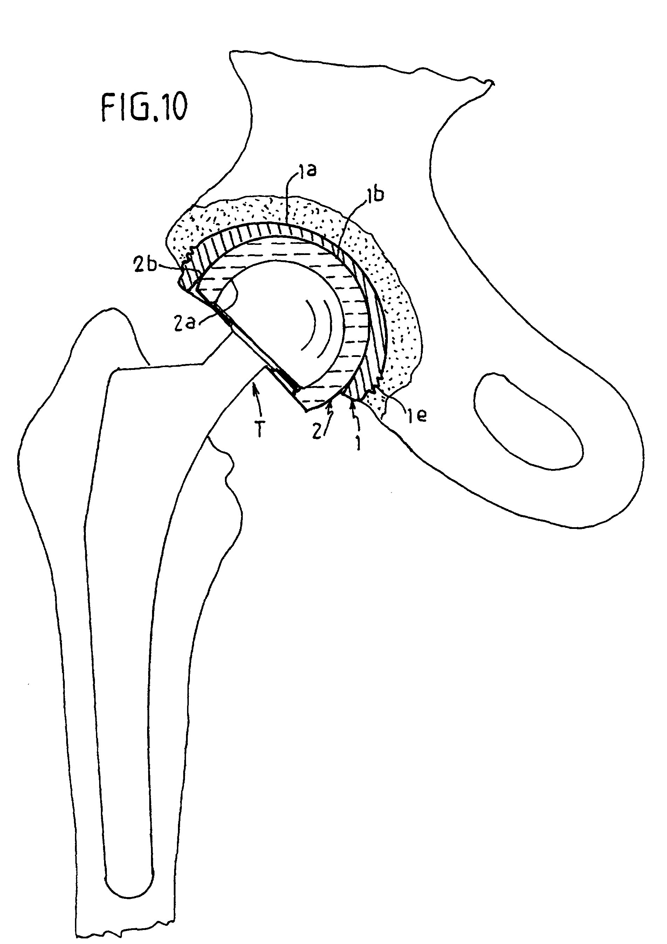

- la figure 10 montre le montage de l'implant cotyloïdien selon les caractéristiques de l'invention dans la cavité cotyloïdienne de l'os iliaque.

D'une manière connue, l'implant cotyloïdien comprend une cupule (1) destinée à être impactée dans la cavité cotyloïdienne de l'os iliaque. Cette cupule (1) est réalisée dans un matériau biocompatible tel que du titane, du chrome-cobalt, de l'acier inoxydable, ...., susceptible d'être revêtu ou non d'un revêtement du type hydroxyapatique de calcium, sans pour cela exclure d'autres matériaux métalliques parfaitement connus et utilisés dans ce domaine technique par l'homme du métier. La cupule (1) est destinée à recevoir un noyau ou insert (2) notamment en polyéthylène. Le noyau (2) est destiné à recevoir, avec capacité de rétention, une tête fémorale d'articulation (T). La cupule (1) a une forme générale hémisphérique.

Le noyau (2) est largement plus qu'hémisphérique pour satisfaire la fonction de rétentivité.

La surface externe (1a) de la cupule (1) est délimitée par une enveloppe hémisphérique dont le centre (O2) est décalé vers l'extérieur par rapport au centre (O1) de la surface interne (1b) de ladite cupule. Le centre de rotation (O1) de la surface interne de la cupule est confondu avec les centres de courbure de la surface interne (2a) et la surface externe (2b) du noyau (2) correspondant au centre de rotation de la tête fémorale (T). Les différents centres (O1) et (O2) sont disposés en alignement selon l'axe de symétrie (X - X') du noyau et de la cupule. Ce décalage des centres (O1) et (O2) correspond à la médialisation.

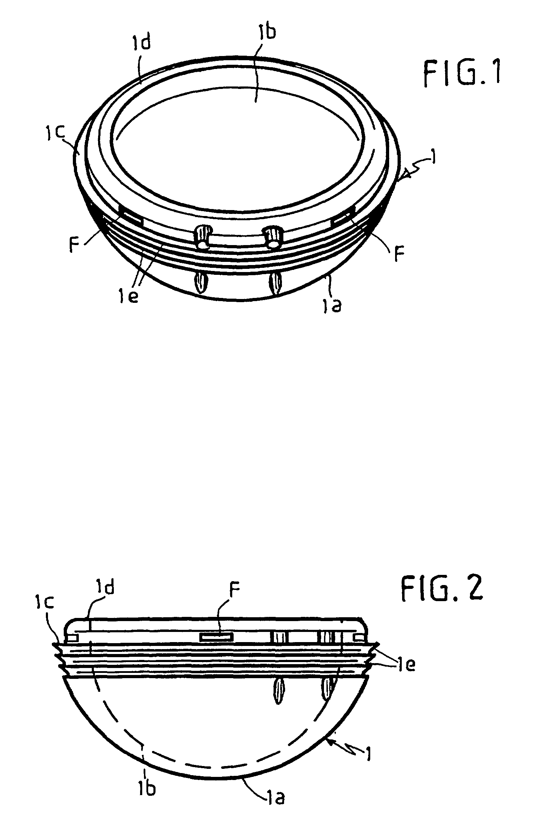

Compte tenu de ces dispositions et comme le montre notamment la figure 2, l'épaisseur de la cupule métallique (1) croit en direction de son bord annulaire (1c) situé dans un plan horizontal perpendiculaire à l'axe de symétrie (X - X'). Le noyau (2) est d'épaisseur constante, compte tenu de la concentricité des faces interne (2a) et externe (2b).

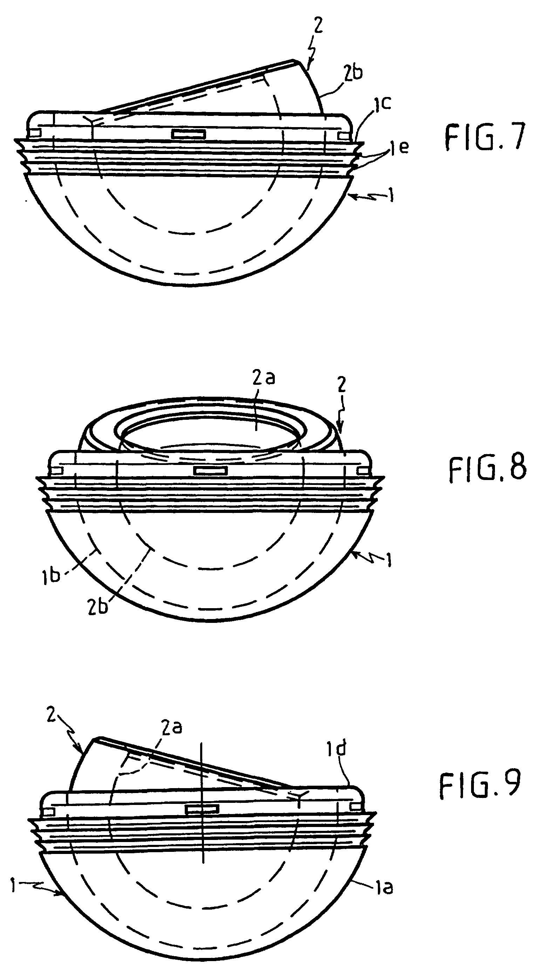

Selon une caractéristique importante de l'invention, le noyau (2) est monté à l'intérieur de la cupule (1) telle que définie, avec capacité de pivotement multidirectionnel. On renvoie par exemple aux figures 7, 8 et 9 montrant différentes orientations du noyau (2) par rapport à la cupule (1).

Compte tenu de la mobilité du noyau (2), la face externe convexe de la cupule (1) constitue très sensiblement une demi-sphère dont le bord circulaire ouvert (1c) présente des agencements de recouvrement partiel du noyau (2) quelle que soit sa position angulaire. Par exemple, ces agencements sont constitués par un rebord cylindrique (1d) formé dans le prolongement de la surface interne hémisphérique (1b) de la cupule et en retrait de sa surface externe (1a). La face externe hémisphérique (1a) de la cupule (1) délimite un angle au centre (α) d'environ 160°, tandis que la face externe du rebord cylindrique (1d) est située dans un plan horizontal parallèle à celui de la face annulaire (1c) de la cupule, en délimitant un angle au centre (β) d'environ 200°. Le noyau mobile (2) délimite une forme externe sphérique d'angle au centre (γ) d'environ 225°. La cavité interne du noyau (2) est cylindro-sphérique.

Selon une autre caractéristique, la face externe de la cupule (1) présente, à partir de son bord annulaire (1c), une série de rainures circulaires (1e), dont les bords externes constituent, en combinaison, une denture fractionnée. Le système de denture, en combinaison avec les 160 degrés de la cupule, assure grâce à l'effet de rétention constitué par le bourrelet cotyloïdien légèrement inférieur au diamètre de l'acetabulum, une parfaite stabilité dans l'acetabulum tant en basculement qu'en rotation.

Il en résulte que la cupule (1) est impactée dans le fond de la cavité cotyloïdienne, sans aucune vis, de sorte que sa surface interne cylindro-sphérique est entièrement lisse et exempte de tout orifice, afin de préserver au maximum le polyéthylène, en évitant le phénomène d'usure et de fluage, compte tenu de sa mobilité multidirectionnelle. A noter que le rebord cylindrique (1d) peut présenter des agencements externes, par exemple sous forme d'un bossage, pour permettre l'engagement de pions, plots ou vis (R) qui apparaissent à l'extérieur de la surface externe de la cupule (1) et des crans (F) aptes à permettre l'engagement de griffes d'ancrage pour un outil préhenseur ou extracteur.

Les avantages ressortent bien de la description, en particulier on souligne et on rappelle :
- augmentation de la stabilité par l'utilisation d'une tête fémorale retenue dans un insert à grand diamètre et par une couverture de l'insert élargie par le fait de la médialisation du centre articulaire ;
- augmentation de la mobilité en supprimant tout effet de came compte tenu de la capacité de déplacement multidirectionnel du noyau dans la cavité interne de la cupule;
- diminution de l'usure du fait de l'absence d'orifice dans son réceptacle ;
- suppression de contraintes d'arrachement du fait de la mobilité de l'insert.

## Revendications

1. Implant cotyloïdien comprenant un noyau (2) notamment en polyéthylène monté dans une cupule métallique (1), ledit noyau (2) recevant une tête fémorale,
- les centres de courbure (O1) de la surface interne et de la surface externe du noyau (2), correspondant au centre de rotation de la tête fémorale, étant confondus avec le centre de la surface interne de la cupule ;
- la surface externe (1a) de la cupule (1) étant délimitée par une enveloppe hémisphérique dont le centre (O2) est décalé vers l'extérieur par rapport au centre (O1) de la surface interne de ladite cupule ; **caractérisé en ce que**
- le noyau (2) est monté à l'intérieur de la cupule (1) avec capacité de pivotement multidirectionnel et qu'il reçoit la tête fémorale avec capacité de rétention.

2. Implant cotyloïdien selon la revendication 1, **caractérisé en ce que** les différents centres (O1) et (O2) sont disposés en alignement selon l'axe de symétrie du noyau (2) et de la cupule (1).

3. Implant cotyloïdien selon la revendication 1, **caractérisé en ce que** la face externe convexe (1a) de la cupule (1) constitue très sensiblement une demi-sphère dont le bord circulaire ouvert présente des agencements de recouvrement partiel du noyau (2), quelle que soit sa position angulaire.

4. Implant cotyloïdien selon la revendication 3, **caractérisé en ce que** les agencements de recouvrement partiel sont constitués par un rebord cylindrique (1d) formé dans le prolongement de la surface interne hémisphérique de la cupule (1) et en retrait de sa surface externe.

5. Implant cotyloïdien selon l'une quelconque des revendications 1 et 4, **caractérisé en ce que** la face externe de la cupule (1) délimite un angle au centre (α) d'environ 160°, tandis que la face interne du rebord cylindrique (1d) est située dans un plan horizontal parallèle à celui de ladite face externe et délimitant un angle au centre (β) de 200° environ.

6. Implant cotyloïdien selon la revendication 1, **caractérisé en ce que** le noyau mobile (2) délimite une forme externe sphérique d'angle au centre (γ) d'environ 225° et une cavité interne cylindro-sphérique.

7. Implant cotyloïdien l'une quelconque des revendications 1 et 4, **caractérisé en ce que** la face externe de la cupule (1) présente, à partir de son bord supérieur où est formé en retrait le rebord cylindrique (1d), des agencements de positionnement et de stabilité dans la cavité cotyloïdienne de l'os iliaque recevant ladite cupule, tels picots, pions ou vis (R).

8. Implant cotyloïdien selon la revendication 7, **caractérisé en ce que** les agencements sont constitués par une série de rainures circulaires parallèles et fractionnées (1e) dont les bords externes constituent, en combinaison, une denture anti-expulsion, anti-rotation, anti-basculement.

9. Implant cotyloïdien selon la revendication 1, **caractérisé en ce que** la cupule (1) est pleine en délimitant une surface interne cylindro-sphérique entièrement lisse et exempt de tout orifice.

10. Implant cotyloïdien selon la revendication 4, **caractérisé en ce que** le rebord cylindrique (1d) présente des agencements externes (F) aptes à permettre l'engagement de griffes d'ancrage, d'un outil préhenseur ou extracteur, qui apparaissent à l'extérieur de la surface externe de la cupule.

## Patentansprüche

1. Hüftgelenkpfannenimplantat mit einem Einsatz (2), insbesondere aus Polyäthylen, der in einer Metallschale (1) montiert wird, wobei der Einsatz (2) einen Hüftkopf aufnimmt,
- wobei die Krümmungsmittelpunkte (O1 ) der Innenfläche und der Außenfläche des Einsatzes (2), die dem Drehpunkt des Hüftkopfes entsprechen, mit dem Mittelpunkt der Innenfläche der Schale zusammenfallen;
- wobei die Außenfläche (1a) der Schale (1) durch einen halbkreisförmigen Mantel begrenzt wird, dessen Mittelpunkt (O2) gegenüber dem Mittelpunkt (O1) der Innenfläche der Schale nach außen versetzt ist; **dadurch gekennzeichnet, dass** der Einsatz (2) im Innern der Schale (1) montiert wird, wobei er in verschiedene Richtungen gedreht werden kann, und dass er den Hüftkopf aufnimmt, den er halten kann.

2. Hüftgelenkpfannenimplantat nach Anspruch 1, **dadurch gekennzeichnet, dass** die verschiedenen Mittelpunkte (O1) und (O2) entlang der Symmetrieachse des Einsatzes (2) und der Schale (1) ausgerichtet sind.

3. Hüftgelenkpfannenimplantat nach Anspruch 1, **dadurch gekennzeichnet, dass** die konvexe Außenseite (1a) der Schale (1) weitgehend Halbkugetform hat, deren offener Scheibenrand ungeachtet ihrer Winkelposition Anordnungen für eine Teilabdeckung des Einsatzes (2) besitzt.

4. Hüftgelenkpfannenimplantat nach Anspruch 3, **dadurch gekennzeichnet, dass** die Anordnungen für eine Teilabdeckung aus einer zylindrischen Randleiste (1d) bestehen, die in der Verlängerung der halbkugelförmigen Innenfläche der Schale (1) und von ihrer Außenfläche zurückgesetzt gebildet werden.

5. Hüngelenkpfannenimplantat nach einem der vorhergehenden Ansprüche 1 und 4, **dadurch gekennzeichnet, dass** die Außenfläche der Schale (1) einen Winkel im Mittelpunkt (α) von ungefähr 160° begrenzt, während die Innenfläche der zylindrischen Randleiste (1d) in einer Horizontalebene parallel zu der der Außenfläche angeordnet ist, und einen Winkel im Mittelpunkt (β) von ungefähr 200° begrenzt.

6. Hüftgelenkpfannenimplantat nach Anspruch 1, **dadurch gekennzeichnet, dass** der bewegliche Einsatz (2) eine externe, runde Winkelform im Mittelpunkt (γ) von ungefähr 225° und eine zylindersphärische Vertiefung begrenzt.

7. Hüftgelenkpfannenimplantat nach einem der vorhergehenden Ansprüche 1 und 4, **dadurch gekennzeichnet, dass** die Außenfläche der Schale (1) ausgehend von ihrem oberen Rand, an dem zurückgesetzt die zylindrische Randleiste gebildet wird, Anordnungen für die Positionierung und die Stabilität im Hüftgelenkpfannenhohlraum des Hüftknochens besitzt, der die Schale aufnimmt, wie Stifte, Metallstücke oder Schrauben (R).

8. Hüftgelenkpfannenimplantat nach Anspruch 7, **dadurch gekennzeichnet, dass** die Anordnungen aus einer Reihe kreisförmiger, paralleler und fraktionierter Einkerbungen (1e) bestehen, deren Außenkanten aus einer Kombination von Anti-Expulsions-, Anti-Rotations- und Anti-Kipp-Verzahnung bestehen.

9. Hüftgelenkpfannenimplantat nach Anspruch 1, **dadurch gekennzeichnet, dass** die Schale (1) massiv ist und eine zylindersphärische Innenfläche begrenzt, die vollkommen glatt ist, und keine Öffnung aufweist.

10. Hüftgelenkpfannenimplantat nach Anspruch 4, **dadurch gekennzeichnet, dass** die zylindrische Randleiste (1d) externe Anordnungen (F) besitzt, die das Eingreifen von Verankerungsklammern, einer Greifvorrichtung oder eines Extraktors ermöglichen, die außen an der Außenfläche der Schale angebracht sind.

## Claims

1. Acetabular implant, comprising an insert (2), in particular of polyethylene, mounted in a metal cup (1), said insert (2) receiving a femoral head
- the centres of curvature (O1) of the inner surface and of the outer surface of the insert (2), corresponding to the centre of rotation of the femoral head, being coincident with the centre of the inner surface of the cup;
- the outer surface (1a) of the cup (1) being delimited by a hemispherical envelope whose centre (O2) is offset outwards in relation to the centre (O1) of the inner surface of said cup; **characterized in that**
- the insert (2) is mounted inside the cup (1) with a capacity for multi-directional pivoting, and **in that** it receives the femoral head with a capacity for retention.

2. Acetabular implant according to Claim 1, **characterized in that** the different centres (O1) and (O2) are arranged in alignment according to the axis of symmetry of the insert (2) and of the cup (1).

3. Acetabular implant according to Claim 1, **characterized in that** the convex outer face (1a) of the cup (1) constitutes very substantially a hemisphere whose open circular edge has arrangements for partial covering of the insert (2) irrespective of its angular position.

4. Acetabular implant according to Claim 3, **characterized in that** the arrangements for partial covering consist of a cylindrical shoulder (1d) formed in the continuation of the hemispherical inner surface of the cup (1) and set back from its outer surface.

5. Acetabular implant according to either of Claims 1 and 4, **characterized in that** the outer face of the cup (1) delimits an angle to the centre (α) of approximately 160°, while the inner face of the cylindrical shoulder (1d) is situated in a horizontal plane parallel to that of said outer face and delimiting an angle to the centre (β) of approximately 200°.

6. Acetabular implant according to Claim 1, **characterized in that** the movable insert (2) delimits a spherical outer shape with an angle to the centre (γ) of approximately 225° and an internal cylindrical-spherical cavity.

7. Acetabular implant according to either of Claims 1 and 4, **characterized in that**, starting from its upper edge where the cylindrical shoulder (1d) is formed in a set-back position, the outer face of the cup (1) has arrangements for positioning and stability in the acetabular cavity of the iliac bone receiving said cup, such as spikes, studs or screws (R).

8. Acetabular implant according to Claim 7, **characterized in that** the arrangements consist of a series of parallel and fractionated circular grooves (1e) whose outer edges constitute, in combination, a toothing which provides for anti-expulsion, anti-rotation and anti-tilting.

9. Acetabular implant according to Claim 1, **characterized in that** the cup (1) is solid and delimits an internal cylindrical-spherical surface which is entirely smooth and without any orifice.

10. Acetabular implant according to Claim 4, **characterized in that** the cylindrical shoulder (1d) has outer arrangements (F) which permit engagement of anchoring claws, a gripper or extractor tool, which appear on the outside of the outer surface of the cup.
